# FASCICULE DE BREVET EUROPEEN

(11) **EP 2 424 660 B1**
(45) Date de publication et mention de la délivrance du brevet: **16.07.2014**
(21) Numéro de dépôt: 10723164.9
(22) Date de dépôt: 23.04.2010
(51) Int. Cl.: B01J 27/188, B01J 37/02, C25B 11/04, H01M 4/92, B01J 23/652, B01J 23/89, B01J 35/00, B01J 31/18, C25B 11/12

(54) **CATALYSEUR ET PROCEDE D'OXYDATION ELECTROCHIMIQUE DU METHANE**
KATALYSATOR UND VERFAHREN ZUR ELEKTROCHEMISCHEN OXIDATION VON METHAN
CATALYST AND METHOD FOR THE ELECTROCHEMICAL OXIDATION OF METHANE

(30) Priorité: 28.04.2009 FR 0902054
(43) Date de publication de la demande: 07.03.2012
(73) Titulaire: Centre National de la Recherche Scientifique (CNRS), 75016 Paris (FR); Université Paris Sud XI, 91405 Orsay (FR)
(72) Inventeur: LU, Yu-Wei, F-91400 Orsay (FR)
(74) Mandataire: Noel, Chantal Odile
(86) Numéro de dépôt international: PCT/FR2010/000328
(87) Numéro de publication internationale: WO 2010/125252

(56) Documents cités:
- EP-A- 0 616 847
- EP-A- 1 569 290
- CN-A- 1 624 963
- BORZENKO M I ET AL: "Platinization assisted by Keggin-type heteropolytungstates" ELECTROCHIMICA ACTA, ELSEVIER SCIENCE PUBLISHERS, BARKING, GB, vol. 48, no. 25-26, 15 novembre 2003 (2003-11-15), pages 3797-3804, XP004463762 ISSN: 0013-4686
- A. KUHN, N. MANO, C. VIDAL: "Polyoxometalate modified electrodes: from a monolayer to multilayer structures" JOURNAL OF ELECTROANALYTICAL CHEMISTRY, vol. 462, 1999, pages 187-194, XP002564439

## Description

L'invention concerne un catalyseur, son utilisation pour la conversion électrochimique du méthane en méthanol et pour la conversion électrochimique directe du méthane en CO₂. Elle concerne également une électrode, en particulier de pile à combustible comprenant un tel catalyseur ainsi qu'un procédé de fabrication d'une telle électrode. Elle concerne encore une pile à combustible comprenant ce catalyseur ou cette électrode.

Les piles à combustible permettent de transformer l'énergie chimique d'un combustible en énergie électrique. La plus connue des piles à combustible est la pile à hydrogène, c'est-à-dire dans laquelle le combustible est l'hydrogène. Une telle pile utilise une membrane échangeuse de protons comme électrolyte qui limite la température d'utilisation inférieure à 90°C. Elle est actuellement la seule pile à combustible destinée à une application embarquée, c'est-à-dire, où elle peut être transportée, comme pour les automobiles, la production et la cogénération résidentielle ou tertiaire, les piles miniatures à hydrogène pour téléphones portables, ordinateurs portables, caméscopes, etc...

Par ailleurs, comme source d'énergie, le méthane est utilisé en tant que combustible pour des automobiles.

L'utilisation du gaz naturel produit du CO₂ qui est un gaz à effet de serre.

En revanche, l'utilisation de méthane provenant de la fermentation naturelle de végétaux n'augmente pas la quantité de CO₂ dans l'air et respecte donc plus l'environnement.

Le méthane, en provenance de la fermentation naturelle des déchets végétaux, constitue une source d'énergie renouvelable, propre et abondant. C'est le seul combustible naturel facile à obtenir sans aucune transformation avant son utilisation. Cette qualité rend son utilisation intéressante, bien plus que celle d'hydrogène et du méthanol.

Le méthane est une molécule totalement synthétique et son oxydation est difficile à basse température.

Une telle oxydation nécessite l'emploi d'un catalyseur.

Cependant, jusqu'à présent, aucun catalyseur connu ne permet la conversion directe du méthane en CO₂, et même la conversion du méthane en méthanol par un procédé électrochimique.

On connaît également les hétéropolyanions de la famille de Keggin (par exemple : H₄SiW₁₂O₄₀) et de la famille de Dawson (par exemple : K₆P₂W₁₈O₆₂) qui sont utilisés comme catalyseurs de réduction électrochimique de H⁺ en H₂ et de l'oxygène en H₂O. Ces hétéropolyanions sont également connus pour être des catalyseurs de réduction électrochimique des nitrates et des nitrites en azote.

Ces hétéropolyanions sont décrits par exemple dans Borzenko et al. "Platinization assisted by keggin-type heteropolytungstates" ELECTROCHIMICA ACTA, ELSEVIER SCIENCE PUBLISHERS, BARKING, GB, vol. 48, no. 25-26, 15 novembre 2003 (2003-11-15), pages 3797-3804, XP004463762 ISSN: 0013-4686 et KUHN et al. "Polyoxometalate modified électrodes: from a monolayer to multilayer structures" JOURNAL OF ELECTROANALYTICAL CHEMISTRY, vol. 462, 1999, pages 187-194, XP002564439.

D'autres catalyseurs contenant du tungstène sont décrits dans la demande de brevet CN 1 624 963 A. EP 1 569 290 A décrit des catalyseurs d'oxydation réduction électrochimique.

Cependant, ces catalyseurs ne permettent pas d'oxyder le méthane en méthanol ou même en CO₂.

De la même façon, les composés du platine de degré d'oxydation II sont connus pour la réduction de H⁺ en H₂ ou l'oxydation de H₂ en H⁺ par voie électrochimique, ils se transforment très vite en platine de degré d'oxydation zéro. Dans tous les cas, ces catalyseurs sont inactifs, pour l'oxydation du méthane en méthanol ou du méthane en CO₂.

D'autres catalyseurs tels que le ruthénium, en particulier complexé par des ligands, sont connus pour des réactions d'hydrogénation des molécules organiques. Les composés de Ni de degré d'oxydation II, et les composés de cobalt de degré d'oxydation II, sont connus pour leur utilisation pour la réduction de H⁺ en H₂, le fer de degré d'oxydation II est également connu en tant que catalyseur de réduction de l'oxygène moléculaire, mais ils sont inefficaces pour la conversion du méthane en méthanol ou du méthane en CO₂.

Les composés de l'ion Ag de degré d'oxydation I, ne sont pas considérés comme des catalyseurs.

L'invention vise à fournir un catalyseur qui permette l'oxydation du méthane en méthanol ou l'oxydation directe du méthane en CO₂, ce qui permet, dans le cas de la conversion du méthane en CO₂ d'utiliser ce catalyseur, en particulier pour des piles à combustible pour des applications embarquées c'est à dire des applications dans des automobiles, des téléphones portables, des groupes électrogène, des ordinateurs portables, etc... en remplacement des piles à combustible à hydrogène.

A cet effet, l'invention propose un catalyseur caractérisé en ce qu'il comprend un précurseur de platine (II), et optionnellement un précurseur d'ion(s) métallique(s) M, supporté(s) sur des particules d'un hétéropolyanion HPA, et en ce que le précurseur de platine (II) est un précurseur de platine ayant un degré d'oxydation II éventuellement complexé par ligand organique ou inorganique,
- M est un ion métallique choisi parmi Ag⁺, Ru²⁺, Ni⁺, Co²⁺, Fe²⁺ et les mélanges de deux ou plus de ceux-ci,
- l'hétéropolyanion HPA est choisi parmi H₄SiW₁₂O₄₀ et K₆P₂W₁₈O₆₂.

Ces hétéropolyanions sont les plus efficaces des hétéropolyanions parmi H₄SiW₁₂O₄₀, K₃PW₁₂O₄₀, K₃PMo₁₂O₄₀, K₆P₂W₁₈O₆₂, K₆P₂W₁₂Mo₆O₆₂.

Dans un premier mode de réalisation, le catalyseur de l'invention ne comprend pas d'ion(s) métallique(s) M.

Dans un second mode de réalisation, le catalyseur de l'invention comprend un (des) ion(s) métallique(s) M.

Dans tous les modes de réalisation du catalyseur de l'invention, le précurseur de platine (II) est choisi parmi le chlorure de platine de formule PtCl₂, le chlorure de bipyridinoplatinium ((Bipy)PtCl₂) de formule suivante : 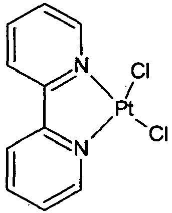 et le chlorure de tétraaminoplatinium de formule Pt(NH₃)₄Cl₂.

Egalement dans tous les modes de réalisation du catalyseur de l'invention, de préférence le précurseur de platine (II) est le (Bipy)PtCl₂ ou le Pt(NH₃)₄Cl₂.

Dans le second mode de réalisation du catalyseur de l'invention, de préférence le précurseur d'ion(s) métallique(s) est un précurseur d'ion Ag⁺.

Toujours dans tous les modes de réalisation de l'invention, l'hétéropolyanion HPA préféré est K₆P₂W₁₈O₆₂ et H₄SiW₁₂O₄₀.

L'invention propose aussi l'utilisation du catalyseur de l'invention selon ces deux modes de réalisation, pour la conversion du méthane en méthanol.

L'invention propose également l'utilisation du catalyseur de l'invention selon le second mode de réalisation pour la conversion directe du méthane en CO₂.

L'invention propose encore une électrode, en particulier pour pile à combustible, caractérisée en ce qu'elle comprend un support en un matériau conducteur électronique sur au moins une surface duquel un catalyseur de l'invention selon le second mode de réalisation est déposé.

De préférence, le matériau conducteur électronique est choisi parmi du carbone vitreux massif, un tissu de carbone, un feutre de carbone et une éponge de titane métallique.

L'invention propose également un procédé de fabrication d'une électrode selon l'invention caractérisé en ce qu'il comprend les étapes suivantes :
(a) mise en solution d'un hétéropolyanion HPA choisi parmi H₄SiW₁₂O₄₀ et K₆P₂W₁₈O₆₂ dans un solvant choisi parmi un alcool linéaire ou ramifié en C₁ à C₄, un mélange d'alcools linéaires ou ramifiés en C₁ à C₄, et un mélange d'eau et d'au moins un alcool linéaire ou ramifié en C₁ à C₄,
(b) dépôt de la solution obtenue à l'étape (a) sur au moins une surface d'un support en un matériau conducteur électronique,
(c) évaporation du solvant de la solution déposée à l'étape (b),
(d) pulvérisation, sur la surface recouverte d'hétéropolyanion obtenu à l'étape (c), d'une solution comprenant un précurseur de platine de degré d'oxydation II, éventuellement complexé par un ligand organique, ou inorganique, et un précurseur d'ion(s) métallique(s) M choisi(s) parmi Ag⁺, Ru²⁺, Co²⁺, Ni²⁺, Fe²⁺ et les mélanges de deux ou plus de ceux-ci, dans un solvant choisi parmi de l'eau, un mélange d'eau et d'au moins un alcool en C₁ à C₄ linéaire ou ramifié, et un mélange d'alcools en C₁ à C₄ linéaires ou ramifiés,
(e) évaporation du solvant de la solution pulvérisée à l'étape (d).

De préférence, à l'étape (a) et à l'étape (c) le solvant est l'isopropanol.

Egalement de préférence le matériau conducteur électronique est choisi parmi du carbone vitreux massif, un feutre ou un tissu de carbone et une éponge de titane métallique.

Toujours de préférence, le précurseur de platine de degré d'oxydation II est choisi parmi PtCl₂, (Bipy)PtCl₂ et Pt(NH₃)₄Cl₂.

Plus préférablement, le précurseur de platine de degré d'oxydation II est choisi parmi le (Bipy)PtCl₂ et le Pt(NH₃)₄Cl₂.

Encore de préférence, le précurseur d'ion(s) métallique(s) M est un précurseur d'ion(s) Ag⁺.

Toujours de préférence, l'hétéropolyanion est K₆P₂W₁₈O₆₂ ou H₄SiW₁₂O₄₀.

Egalement de préférence, le matériau conducteur électronique est une éponge de titane métallique ou un feutre ou un tissu de carbone.

L'invention propose aussi un procédé de transformation du méthane en méthanol caractérisé en ce qu'il comprend une étape d'utilisation d'un catalyseur de l'invention selon le premier mode de réalisation.

L'invention propose également un procédé d'oxydation directe du méthane en CO₂ caractérisé en ce qu'il comprend une étape de mise en contact du méthane CH₄ avec un catalyseur de l'invention selon le second mode de réalisation.

Enfin, l'invention propose une pile à combustible caractérisée en ce qu'elle comprend un catalyseur de l'invention selon le second mode de réalisation ou une électrode selon l'invention ou une électrode obtenue par le procédé selon l'invention.

L'invention sera mieux comprise et d'autres caractéristiques et avantages de celle-ci apparaîtront plus clairement à la lecture de la description explicative qui suit.

Le catalyseur selon l'invention est un catalyseur constitué d'un support, lui-même constitué de particules d'un hétéropolyanion, noté HPA, de la famille de Keggin ou de Dawson, sur lequel des particules d'un précurseur de platine de degré d'oxydation II, et optionnellement un précurseur d'ion(s) métallique(s) M sont déposés.

L'hétéropolyanion de Keggin a la formule H₄SiW₁₂O₄₀ et l'hétéropolyanion de Dawson a la formule K₆P₂W₁₈O₆₂.

Ainsi, dans un premier mode de mise oeuvre, le catalyseur de l'invention est constitué du support hétéropolyanion de Keggin ou de Dawson sur lequel est déposé du platine de degré d'oxydation II, noté également Pt(II) ou platine (II). Ces notations représentent aussi bien un précurseur dans lequel le platine a le degré d'oxydation II sous forme de sel, qu'un précurseur dans lequel le platine est complexé par un ou plusieurs ligands.

Dans l'invention, on préfère utiliser, en tant que précurseur de platine de degré d'oxydation II, le chlorure de platine, de formule PtCl₂, ou le chlorure de tétraaminoplatinium de formule Pt(NH₃)₄Cl₂, ou encore un complexe de platine qui est le (Bipy)PtCl₂ de formule suivante :

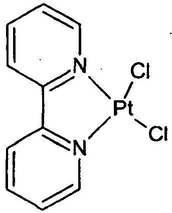

Avec le catalyseur de l'invention, le méthane se fixe par liaison sur le platine qui est déposé sur l'hétéropolyanion pour former du méthanol par voie électrochimique.

Dans un mode de réalisation préféré, le catalyseur de l'invention comprend de plus un précurseur d'ion(s) métallique(s) noté(s) M. Ce (ces) ion(s) métallique(s) est (sont) de préférence choisi(s) parmi le(s) ion(s) Ag⁺, Ru²⁺, Co²⁺, Ni⁺, Fe²⁺ et les mélanges de deux ou plus de ceux-ci.

On préfère tout particulièrement utiliser des ions Ag⁺ pour former le catalyseur de l'invention.

Le nombre d'ions platine (II) et d'ion(s) métallique(s) M dépend de l'hétéropolyanion et de la valence de l'ion métallique. Ainsi, lorsque l'ion métallique est Ag⁺, et que l'hétéropolyanion, HPA, est l'hétéropolyanion de la famille de Keggin, le nombre d'ions Ag⁺ déposés est deux fois supérieur au nombre d'ions Pt²⁺ déposés.

En revanche, lorsque l'ion métallique est Ru²⁺, ou Co²⁺ ou Ni²⁺ ou Fe²⁺, le nombre de ces ion(s) métallique(s) déposé(s) est égal au nombre d'ion(s) Pt²⁺ déposé(s).

Lorsque l'hétéropolyanion est un hétéropolyanion de Dawson, et que l'ion métallique est Ag⁺, le nombre d'ions Ag⁺ déposés sur l'hétéropolyanion est de quatre ions Ag⁺ pour un ion Pt^{2+.}

Lorsque l'ion métallique est Ru²⁺ ou Co²⁺ ou Ni²⁺ ou Fe²⁺, le nombre d'ions métalliques déposés sur l'hétéropolyanion est de deux ions métalliques pour un ion Pt²⁺.

Avec ce second mode de réalisation, qui est un mode de réalisation préféré, du catalyseur de l'invention, on obtient soit la transformation du méthane en méthanol, soit la conversion directe du méthane en CO₂. Dans cette réaction de conversion directe, le méthane se fixe par liaison sur le platine pour donner le méthanol, le méthanol étant ensuite oxydé par les ions métalliques M pour donner CO₂.

De préférence, dans cette réaction de conversion directe du méthane en CO₂, on utilise des ions métalliques Pt²⁺ et des ions métalliques tels que définis ci-dessus et en tant que support un hétéropolyanion de Dawson qui a une réactivité plus forte que l'hétéropolyanion de Keggin. Cependant, l'hétéropolyanion de Dawson est insoluble dans l'alcool, ce qui le rend plus difficile à mettre en oeuvre dans une pile à combustible. C'est pourquoi, dans certains cas, il peut être préférable d'utiliser l'hétéropolyanion de Keggin qui a une réactivité plus faible mais qui est plus facile à mettre en solution car soluble dans un alcool.

Le catalyseur de l'invention permet d'utiliser très peu de platine, ce qui est encore un de ses avantages.

En effet, des quantités déposées de deux microgrammes par centimètre carré de surface de l'hétéropolyanion sont suffisantes pour obtenir le catalyseur de l'invention, selon le premier ou selon le second mode de réalisation.

La quantité de platine utilisée est de un ion Pt II pour 50 à 100 ions de HPA. Plus il y a du platine, plus la réaction d'oxydation est rapide. Si ce rapport est inférieur à 10, l'oxydation partielle du CH₄ est favorisée.

Quant à la quantité d'ions métalliques déposés, elle peut varier suivant le degré d'oxydation de ces ions. L'ion métallique sert à conserver la couche de l'hétéropolyanion. Il s'échange avec les contre-ions de l'HPA jusqu'à la neutralité totale des charges portées de l'HPA.

Ainsi, le catalyseur selon le second mode de réalisation de l'invention est particulièrement utile pour la fabrication d'une électrode, en particulier d'une pile à combustible. Cette électrode est constituée d'un support en un matériau conducteur électronique sur au moins une surface duquel un catalyseur selon l'invention est déposé.

Le matériau conducteur électronique préféré est choisi parmi le carbone vitreux massif, un tissu de carbone, un feutre de carbone et une éponge de titane métallique.

De préférence, pour une utilisation en tant qu'électrode de pile à combustible, le support en matériau conducteur électronique préféré est une éponge de titane métallique.

Cependant, un feutre de carbone ou un tissu de carbone sont également préférés.

L'électrode selon l'invention, en particulier pour pile à combustible, peut être fabriquée par un procédé qui comprend les étapes de mise en solution de l'hétéropolyanion dans un alcool linéaire ou ramifié en C₁ à C₄, ou dans un mélange d'alcool linéaire ou ramifié en C₁ à C₄, ou encore dans un mélange d'eau et d'au moins un alcool linéaire ou ramifié en C₁ à C₄, de l'hétéropolyanion choisi.

De préférence, on utilisera, comme solvant, de l'isopropanol.

Puis la solution obtenue est déposée, par exemple par pulvérisation, sur au moins une surface d'un support en matériau conducteur électronique et le solvant est évaporé, le solvant est avantageusement évaporé librement à température ambiante.

De préférence, le support en un matériau conducteur électronique est du carbone vitreux massif ou des tissus ou des feutres de carbone vitreux. Le plus préférablement, le support est une éponge de titane métallique.

Puis, une solution composée du précurseur de platine de degré d'oxydation II, de préférence PtCl₂, ou (Bipy)PtCl₂ ou Pt(NH₃)₄Cl₂, et éventuellement de(s) l'ion(s) métallique(s) M choisi parmi Ag⁺, Ru²⁺, Ni²⁺, Co²⁺, Fe²⁺ dans un solvant est pulvérisée sur la surface du support en un matériau conducteur électronique recouvert de l'hétéropolyanion HPA. Le solvant du précurseur de platine (II), et éventuellement de(s) l'ion(s) métallique(s) M est choisi parmi l'eau ou un mélange d'eau et d'au moins un alcool en C₁ à C₄ linéaire ou ramifié, un alcool en C₁ à C₄ linéaire ou ramifié, ou un mélange de deux ou plus alcools en C₁ à C₄ linéaires ou ramifiés

De préférence, ce solvant est l'isopropanol, c'est-à-dire le même solvant que celui ayant servi à solubiliser l'hétéropolyanion HPA.

Ce solvant est ensuite évaporé, avantageusement librement à l'air à la température ambiante.

Bien entendu, comme le reconnaîtra l'homme de l'art, la surface du matériau conducteur électronique doit être parfaitement propre pour éviter toute contamination du ou des catalyseurs.

De préférence, on utilisera, en tant que précurseur du platine de degré d'oxydation II, le PtCl₂, le (Bipy)PtCl₂ ou le Pt(NH₃)₄Cl₂.

Lorsqu'un (des) ion(s) métallique(s) est (sont) de plus présent(s) dans le catalyseur de l'invention, cet (ces) ion(s) métallique(s) est (sont) de préférence des ions Ag⁺.

Quant à l'hétéropolyanion HPA, il est également, comme pour le catalyseur de l'invention, de préférence un hétéropolyanion de la famille de Dawson ou de Keggin.

L'invention propose encore une pile à combustible qui comprend un catalyseur selon l'invention tel que décrit ci-dessus, ou une électrode selon l'invention telle que décrite ci-dessus, ou une électrode obtenue par le procédé selon l'invention tel que décrit ci-dessus.

Le catalyseur de l'invention, ou l'électrode de l'invention ou l'électrode obtenue par le procédé de l'invention, lorsque le catalyseur ne comprend pas d'ion(s) métallique(s) M, est particulièrement destiné à la mise en oeuvre d'un procédé de transformation du méthane en méthanol.

Lorsque le catalyseur de l'invention, ou lorsque l'électrode de l'invention ou lorsque l'électrode obtenue par le procédé de l'invention comprenant ce catalyseur, est un catalyseur dans lequel un (des) ion(s) métallique(s) M est (sont) présent(s), de préférence, ce catalyseur et cette électrode sont particulièrement appropriés pour la mise en oeuvre d'un procédé d'oxydation directe du méthane en CO₂, en particulier lorsque le(s) ion(s) métallique(s) est (sont) Ag⁺.

L'invention concerne également tous dispositifs, en particulier mobiles, qui comprendraient une pile à combustible selon l'invention ou qui utiliserait un catalyseur ou une électrode selon l'invention ou une électrode obtenue par le procédé selon l'invention.

De tels dispositifs, à titre d'exemples, sont des automobiles, des téléphones portables ou des ordinateurs portables ou encore des groupes électrogènes.

Afin de mieux faire comprendre l'invention, on va en décrire maintenant, à titre d'exemple purement illustratif et non limitatif, plusieurs exemples de réalisation.

### Exemple 1 :

Préparation d'une électrode pour étude analytique de l'oxydation du méthane.

Deux µL d'une solution d'un hétéropolyanion de Keggin (H₄SiW₁₂O₄₀) à une concentration de 10mg par mL dans de l'isopropanol ou d'un hétéropolyanion de Dawson (K₆P₂W₁₈O₆₂) à une concentration de 5mg par mL dans de l'eau-isopropanol sont déposés sur une surface polie de carbone vitreux de 3 millimètres de diamètre. Après évaporation du solvant isopropanol, 2 µL d'un mélange comprenant un précurseur d'ions platine de degré d'oxydation II ici (PtCl₂, Pt(Bipy)Cl₂ ou Pt(NH₃)₄Cl₂) à une concentration de 2.10⁻⁵ M et un précurseur d'ions métalliques Ag⁺ sous la forme de AgNO₃ à une concentration 0,08M sont déposés. Le solvant utilisé est de l'eau ou mélange l'eau-isopropanol. Ce solvant est ensuite évaporé. Sous la lumière de Néon, on voit une aréole colorée de plusieurs couleurs quand le film est mince avec H₄SiW₁₂O₄₀ ou blanchâtre avec K₆P₂W₁₈O₆₂

### Exemple 2 :

Test de l'électrode obtenue à l'exemple 1 pour la conversion directe du méthane en CO₂.

L'électrode obtenue à l'exemple 1 est plongée dans une solution tampon pH 3 de Na₂SO₄/H₂SO₄ et cyclée entre 0,9 et 1,7 volt par rapport à une électrode au calomel saturé (ECS) sous argon jusqu'à ce que le courant se stabilise. Il faut environ 30 minutes à 1 heure. Puis la solution est saturée par diffusion de méthane à la surface. La saturation est lente. La catalyse peut durer plus de 3 heures.

Ce test est effectué à température ambiante, c'est-à-dire à une température comprise entre 15°C et 25°C.

### Exemple 3 :

Préparation d'une électrode de grande surface avec le catalyseur selon l'invention.

Une solution d'un hétéropolyanion de Keggin (H₄SiW₁₂O₄₀) à une concentration de 10mg par mL dans de l'isopropanol ou d'un hétéropolyanion de Dawson (K₆P₂W₁₈O₆₂) à une concentration de 5mg par mL dans de l'eau-isopropanol est pulvérisée sur une surface de carbone vitreux poli de 3cm².

L'isopropanol est évaporé puis un mélange du précurseur de platine de degré d'oxydation II à une concentration 2.10⁻⁵M et du précurseur d'ions métalliques Ag⁺ à une concentration 8.10⁻² M est projeté sur la surface de carbone vitreux sur laquelle l'hétéropolyanion a été déposé.

Le solvant, qui est également ici l'eau-isopropanol, est ensuite évaporé.

### Exemple 4 :

Test de l'électrode de l'exemple 3 pour la conversion directe du méthane en CO₂.

L'électrode obtenue à l'exemple 3 est immergée dans le compartiment d'une cellule à deux compartiments remplis de solution tampon pH 3 de Na₂SO₄ 0,1M. L'autre compartiment de la cellule comprend une électrode en platine massif. Dès le contact du méthane avec le catalyseur, la différence de potentiel entre l'électrode de platine massif (pôle positif) et l'électrode selon l'invention (pôle négatif) croît jusqu'à 0,4 volt, ce qui montre bien la fixation du CH₄ sur le catalyseur.

**Tableau 1 : Différence de potentiel entre le pôle positif (Pt) et le pôle négatif (CV sans ou avec le catalyseur PtM(HPA))**

| | Sans | Avec |
|---|---|---|
| Air | 0,350V | 0,151 V |
| Ar | 0,432V | 0,152V |
| CH4 | 0,432 V | 0,428V |

| | | |
|---|---|---|
| Conditions expérimentales : 0,1M Na₂SO₄/H₂SO₄ pH 3, ECS, Pt, M=Ag, HPA = H₄SiW₁₂O₄₀. | | |

Si les deux électrodes sont reliées par un conducteur en cuivre et un ampèremètre en série, un courant faible circule dans la cellule. La présence de CO₂ dans la solution (détecté par CPG-SM) confirme la conversion directe du méthane en CO₂.

Là encore, ce test est réalisé à température ambiante.

### Exemple 5 : Préparation d'une électrode pour la pile à combustible au méthane

Sur une éponge de titane de 25cm², 1 mL d'une solution d'un hétéropolyanion de Keggin (H₄SiW₁₂O₄₀) à une concentration de 10mg par mL dans de l'isopropanol ou d'un hétéropolyanion de Dawson (K₆P₂W₁₈O₆₂) à une concentration de 5mg par mL dans de l'eau-isopropanol est projetée. L'isopropanol est évaporé. Puis une solution contenant 0,1 mL d'une solution contenant des ions platines de degré d'oxydation II à une concentration 10⁻⁴M ajoutés sous la forme du précurseur, PtCl₂, Pt(Bipy)Cl₂ ou Pt(NH₃)₄Cl₂, et 0,1 mL d'une solution contenant des ions métalliques Ag⁺ à une concentration de 10⁻¹ M, dans 0,3 mL d'isopropanol est déposé sur la surface de la mousse de titane recouverte de l'hétéropolyanion.

Cette électrode est montée dans une pile à combustible à hydrogène. Cette pile, bien que n'ayant pas de sortie pour l'évacuation du CO₂ produit par la combustion du méthane, après une période d'induction, est opérationnelle. L'intensité de courant à circuit fermé reste stable durant plus de 5 minutes puis elle diminue lentement jusqu'à l'arrêt dû au CO₂ cumulé dans la pile. La performance n'a pas pu être déterminée dans ces conditions.

Cet exemple montre que le catalyseur de l'invention et l'électrode de l'invention peuvent être utilisés dans une pile à combustible fonctionnant au méthane à température ambiante.

De manière générale, les exemples ci-dessus montrent que le catalyseur de l'invention permet la conversion du méthane en méthanol ou en CO₂.

Des tests réalisés à 80°C montrent que le catalyseur de l'invention est stable à cette température et permet toujours de réaliser la conversion du méthane en méthanol ou en CO₂.

## Revendications

1. Catalyseur **caractérisé en ce qu'**il comprend un précurseur de platine (Il), et optionnellennent un précurseur d'ion(s) métallique(s) M, supporté(s) sur des particules d'un hétéropolyanion HPA,
et **en ce que** :
le précurseur de platine (II) est un précurseur de platine ayant un degré d'oxydation 2+, éventuellement complexé par ligand organique ou inorganique,
- M est un ion métallique choisi parmi Ag⁺, Ru²⁺, Ni²⁺, Co²⁺, Fe²⁺ et les mélanges de deux ou plus de ceux-ci,
- l'hétéropolyanion HPA est choisi parmi E₄SiW₁₂O₄₀ et K₆P₂W₁₈O₆₂, de préférence l'hétéropolyanion HPA est K₆P2W₁₈O₆₂.

2. Catalyseur selon la revendication 1 **caractérisé en ce qu'**il ne comprend pas d'ion(s) métallique(s) M.

3. Catalyseur selon la revendication 1 **caractérisé en ce qu'**il comprend au moins un ion métallique M.

4. Catalyseur selon la revendication 1 **caractérisé en ce que** le précurseur de platine (II) est choisi parmi le chlorure de platine de formule PtCl₂, 1c chlorure de bipyridinoplatinium, (Bipy)PtCl₂, de formule suivante : 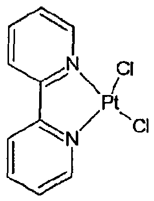 et le chlorure de tétraaminoplatinium de formule Pt(NH₃)₄Cl₂.

5. Catalyseur selon l'une quelconque des revendications précédentes **caractérisé en ce que** le précurseur de platine (II) est le (Bipy)PtCl₂ ou le Pt(NH₃)₄Cl₂.

6. Catalyseur selon l'une quelconque des revendications 1 et 3 à 5 **caractérisé en ce que** le précurseur d'ion(s) métallique(s) est un précurseur d'Ag⁺.

7. Utilisation du catalyseur selon l'une quelconque des revendications précédentes pour la conversion du méthane en méthanol.

8. Utilisation du catalyseur selon l'une quelconque des revendications 1 et 3 à 6 pour la conversion du méthane en CO₂.

9. Electrode, en particulier de pile à combustible, **caractérisée en ce qu'**elle comprend un support en un matériau conducteur électronique sur au moins une surface duquel un catalyseur selon l'une quelconque des revendications 1 et 3 à 6 est déposé.

10. Electrode selon la revendication 9 **caractérisée en ce que** le matériau conducteur électronique est choisi parmi du carbone vitreux massif, un tissu de carbone vitreux, un feutre de carbone vitreux et une éponge de titane métallique.

11. Procédé de fabrication de l'électrode selon la revendication 9 ou 10 **caractérisé en ce qu'**il comprend les étapes suivantes :
(a) mise en solution d'un hétéropolyanion HPA choisi parmi H₄SiW₁₂O₄₀ et K₆P₂W₁₈O₆₂ dans un solvant choisi parmi un alcool linéaire ou ramifié en C₁ à C₄, un mélange d'alcools linéaires ou ramifiés en C₁ à C₄, et un mélange d'eau et d'au moins un alcool linéaire ou ramifié en C₁ à C₄,
(b) dépôt de la solution obtenue à l'étape (a) sur au moins une surface d'un support en un matériau conducteur électronique,
(c) évaporation du solvant de la solution déposée à l'étape (b),
(d) pulvérisation, sur la surface revêtue d'hétéropolyanion obtenue à l'étape (c) d'une solution comprenant un précurseur de platine de degré d'oxydation II, éventuellement complexé par un ligand organique ou inorganique, et un précurseur d'ion(s) métallique(s) M choisi(s) parmi Ag⁺, Ru²⁺, Co²⁺, Ni²⁺, Fe²⁺ et les mélanges de deux ou plus de ceux-ci, dans un solvant choisi parmi de l'eau, un mélange d'eau et d'au moins un alcool en C₁ à C₄ linéaire ou ramifié, un alcool en C₁ à C₄ linéaire ou ramifié et un mélange d'alcools en C₁ à C₄ linéaires ou ramifiés, et
(e) évaporation du solvant de la solution pulvérisée à l'étape (d).

12. Procédé selon la revendication 11 **caractérisé en ce qu'**à l'étape (a) et à l'étape (c) le solvant est l'isopropanol.

13. Procédé selon la revendication 11 ou 12 **caractérisé en ce que** le matériau conducteur électronique est choisi parmi du carbone vitreux massif, un feutre de carbone, un tissu de carbone et une éponge de titane métallique.

14. Procédé selon l'une quelconque des revendications 11 à 13 **caractérisé en ce que** le précurseur de platine de degré d'oxydation II est choisi parmi PtCl₂, (Bipy)PtCl₂ et Pt(NH₃)₄Cl₂.

15. Procédé selon l'une quelconque des revendications 11 à 14 **caractérisé en ce que** le composé de platine de degré d'oxydation II est choisi parmi (Bipy)PtCl₂ et Pt(NH₃)₄Cl₂.

16. Procédé selon l'une quelconque des revendications 11 à 15 **caractérisé en ce que** le(s) ion(s) métallique(s) M du précurseur d'ion(s) métallique(s) est (sont) Ag⁺.

17. Procédé selon l'une quelconque des revendications 11 à 16 **caractérisé en ce que** l'hétéropolyanion est K₆P₂W₁₈O₆₂.

18. Procédé selon l'une quelconque des revendications 11 à 17 **caractérisé en ce que** le matériau conducteur électronique est une éponge de titane métallique ou un feutre ou un tissu de carbone.

19. Pile à combustible **caractérisée en ce qu'**elle comprend un catalyseur selon l'une quelconque des revendications 1 et 3 à 6.

20. Pile à combustible **caractérisée en ce qu'**elle comprend une électrode selon l'une quelconque des revendications 9 ou 10, ou une électrode obtenue par le procédé selon l'une quelconque des revendications 11 à 18.

## Patentansprüche

1. Katalysator, **dadurch gekennzeichnet, dass** er einen Vorläufer von Platin(II) und gegebenenfalls einen Vorläufer von metallischem Ion (metallischen Ionen) M, getragen auf Partikeln eines Heteropolyanions HPA, umfasst
und dass:
der Vorläufer von Platin(II) ein Vorläufer von Platin mit einer Oxidationsstufe 2+, gegebenenfalls mit einem organischen oder anorganischen Liganden komplexiert ist,
- M ein metallisches Ion, ausgewählt aus Ag⁺, Ru²⁺, Ni²⁺, Co²⁺, Fe²⁺ und Gemischen aus zwei oder mehr von diesen, ist,
- das Heteropolyanion HPA ausgewählt ist aus H₄SiW₁₂O₄₀ und K₆P₂W₁₈O₆₂, das Heteropolyanion HPA vorzugsweise K₆P₂W₁₈O₆₂ ist.

2. Katalysator nach Anspruch 1, **dadurch gekennzeichnet, dass** er kein metallisches Ion (keine metallischen Ionen) M umfasst.

3. Katalysator nach Anspruch 1, **dadurch gekennzeichnet, dass** er wenigstens ein metallisches Ion M umfasst.

4. Katalysator nach Anspruch 1, **dadurch gekennzeichnet, dass** der Vorläufer von Platin(II) aus Platinchlorid der Formel PtCl₂, Bipyridinoplatinchlorid(Bipy) PtCl₂ der folgenden Flormel: 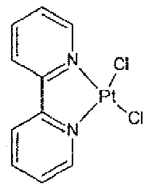 und Tetraaminoplatinchlorid der Formel Pt(NH₃)₄Cl₂ ausgewählt ist.

5. Katalysator nach einem der vorangehenden Ansprüche, **dadurch gekennzeichnet, dass** der Vorläufer von Platin(II) (Bipy)PtCl₂ oder Pt(NH₃)₄Cl₂ ist.

6. Katalysator nach einem der Ansprüche 1 und 3 bis 5, **dadurch gekennzeichnet, dass** der Vorläufer von metallischem Ion (metallischen Ionen) ein Vorläufer von Ag⁺ ist.

7. Verwendung des Katalysators nach einem der vorangehenden Ansprüche für die Umwandlung von Methan in Methanol.

8. Verwendung des Katalysators nach einem der Ansprüche 1 und 3 bis 6 für die Umwandlung von Methan in CO₂.

9. Elektrode, insbesondere einer Brennstoffzelle, **dadurch gekennzeichnet, dass** sie wenigstens einen Träger aus einem elektronenleitenden Material umfasst, wobei auf wenigstens einer Oberfläche desselben ein Katalysator nach einem der Ansprüche 1 und 3 bis 6 abgeschieden ist.

10. Elektrode nach Anspruch 9, **dadurch gekennzeichnet, dass** das elektronenleitende Material aus massivem Glaskohlenstoff, einem Glaskohlenstoffgewebe, einem Glaskohlenstofffilz und einem Schwamm aus metallischem Titan ausgewählt ist.

11. Verfahren zur Herstellung der Elektrode nach Anspruch 9 oder 10, **dadurch gekennzeichnet, dass** es die folgenden Stufen umfasst:
(a) In-Lösung-Bringen eines Heteropolyanions HPA, ausgewählt aus H₄SiW₁₂O₄₀ und K₆P₂W₁₈O₆₂, in einem Lösungsmittel, ausgewählt aus einem linearen oder verzweigten C₁-C₄-Alkohol, einem Gemisch linearer oder verzweigter C₁-C₄-Alkoholen und einem Gemisch aus Wasser und wenigstens einem linearen oder verzweigten C₁-C₄-Alkohol;
(b) Abscheiden der in Stufe (a) erhaltenen Lösung auf wenigstens eine Oberfläche eines Trägers aus elektronenleitendem Material;
(c) Verdampfung des Lösungsmittels der in Stufe (b) abgeschiedenen Lösung;
(d) Aufsprühen auf die Oberfläche, die mit Heteropolyanion überzogen ist, die in Stufe (c) erhalten wurde, einer Lösung, die einen Vorläufer von Platin mit der Oxidationsstufe II, gegebenenfalls komplexiert mit einem organischen oder anorganischen Liganden, und einen Vorläufer von metallischem Ion (metallischen Ionen) M, ausgewählt aus Ag⁺, Ru²⁺, Co²⁺, Ni²⁺, Fe²⁺ und Gemischen aus zwei oder mehreren dieser, in einem Lösungsmittel, ausgewählt aus Wasser, einem Gemisch aus Wasser und wenigstens einem linearen oder verzweigten C₁-C₄-Alkohol, einem linearen oder verzweigten C₁-C₄-Alkohol und einem Gemisch linearer oder verzweigter C₁-C₄-Alkohole, umfasst und
(e) Verdampfung des Lösungsmittels der Lösung, die in Stufe (d) aufgesprüht wurde.

12. Verfahren nach Anspruch 11, **dadurch gekennzeichnet, dass** das Lösungsmittel in Stufe (a) und in Stufe (c) Isopropanol ist.

13. Verfahren nach Anspruch 11 oder 12, **dadurch gekennzeichnet, dass** das elektronenleitende Material aus massivem Glaskohlenstoff, einem Kohlenstofffilz, einem Kohlenstoffgewebe und einem Schwamm aus metallischem Titan ausgewählt wird.

14. Verfahren nach einem der Ansprüche 11 bis 13, **dadurch gekennzeichnet, dass** der Vorläufer von Platin mit der Oxidationsstufe II aus PtCl₂(Bipy)PtCl₂ und Pt(NH₃)₄Cl₂ ausgewählt wird.

15. Verfahren nach einem der Ansprüche 11 bis 14, **dadurch gekennzeichnet, dass** die Verbindung von Platin mit der Oxidationsstufe II aus (Bipy)PtCl₂ und Pt(NH₃)₄Cl₂ ausgewählt wird.

16. Verfahren nach einem der Ansprüche 11 bis 15, **dadurch gekennzeichnet, dass** das metallische Ion (die metallischen Ionen) M des Vorläufers von metallischem Ion (metallischen Ionen) Ag⁺ ist (sind).

17. Verfahren nach einem der Ansprüche 11 bis 16, **dadurch gekennzeichnet, dass** das Heteropolyanion K₆P₂W₁₈O₆₂ ist.

18. Verfahren nach einem der Ansprüche 11 bis 17, **dadurch gekennzeichnet, dass** das elektronenleitende Material ein Schwamm von metallischem Titan oder ein Kohlenstofffilz oder -gewebe ist.

19. Brennstoffzelle, **dadurch gekennzeichnet, dass** sie einen Katalysator nach einem der Ansprüche 1 und 3 bis 6 umfasst.

20. Brennstoffzelle, **dadurch gekennzeichnet, dass** sie eine Elektrode nach einem der Ansprüche 9 oder 10 oder eine Elektrode, erhalten durch das Verfahren nach einem der Ansprüche 11 bis 18, umfasst.

## Claims

1. A catalyst **characterised in that** it comprises a precursor of platinum (II) and optionally a precursor of metal ion or ions M supported on heteropolyanion particles HPA,
and **in that**:
the precursor of platinum (II) is a precursor of platinum having an oxidation state 2+, which may possibly be complexed by an organic or inorganic ligand,
- M is a metal ion selected from Ag⁺, Ru²⁺, Ni²⁺, Co²⁺, Fe²⁺ and mixtures or two or more of these,
- the heteropolyanion HPA is selected from H₄SiW₁₂O₄₀ and K₆P₂W₁₈O₆₂, preferably the heteropolyanion HPA is K₆P₂W₁₈O₆₂.

2. The catalyst according to claim 1 **characterised in that** it does not comprise any metal ion or ions M.

3. The catalyst according to claim 1 **characterised in that** it comprises at least one metal ion M.

4. The catalyst according to claim 1 **characterised in that** the precursor of platinum (II) is selected from platinum chloride having the formula PtCl₂, bipyridinoplatinium chloride (Bipy)PtCl₂ having the following formula: 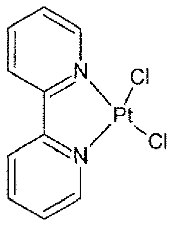 and tetraaminoplatinium chloride of formula Pt(NH₃)₄Cl₂.

5. The catalyst according to any one of the preceding claims **characterised in that** the precursor of platinum (II) is (Bipy)PtCl₂ or Pt(NH₃)₄Cl₂.

6. The catalyst according to any one of claims 1 and 3 to 5 **characterised in that** the precursor of the metal ion(s) is a precursor of Ag⁺.

7. Use of the catalyst according to any one of the preceding claims for the conversion of methane to methanol.

8. Use of the catalyst according to any one of claims 1 and 3 to 6 for the conversion of methane into CO₂.

9. An electrode, in particular of a fuel cell, **characterised in that** it comprises a substrate of an electron-conducting material on at least one surface of which a catalyst according to any one of claims 1 and 3 to 6 has been deposited.

10. An electrode according to claim 9 **characterised in that** the electron-conducting material is selected from massy vitreous carbon, vitreous carbon fabric, a felt of vitreous carbon and a titanium metal sponge.

11. A process for manufacturing the electrode according to claim 9 or 10 **characterised in that** it comprises the following steps:
(a) dissolution of a heteropolyanion HPA selected from H₄SiW₁₂O₄₀ and K₆P₂W₁₈O₆₂ in a solvent selected from a linear or branched C₁ to C₄ alcohol, a mixture of linear or branched C₁ to C₄ alcohols, and a mixture of water and at least one linear or branched C₁ to C₄ alcohol,
(b) deposition of the solution obtained in step (a) onto at least one surface of a substrate of an electron-conducting material,
(c) evaporation of the solvent from the solution deposited in step (b),
(d) spraying of a solution comprising a precursor of platinum having an oxidation state II, possibly complexed by an organic or inorganic ligand, and a precursor of metal ion or ions M selected from Ag⁺, Ru²⁺, Co²⁺, Ni²⁺, Fe²⁺ and mixtures of two or more of these in a solvent selected from water, a mixture of water and at least one linear or branched C₁ to C₄ alcohol, a linear or branched C₁ to C₄ alcohol and a mixture of liner or branched C₁ to C₄ alcohols, onto the surface coated with the heteropolyanion obtained in step (c), and
(e) evaporation of the solvent of the solution sprayed in step (d).

12. The process according to claim 11 **characterised in that** the solvent in stage (a) and stage (c) is isopropanol.

13. The process according to claim 11 or 12 **characterised in that** the electron-conducting material is selected from massy vitreous carbon, a carbon felt, a carbon fabric or a titanium metal sponge.

14. The process according to any one of claims 11 to 13 **characterised in that** the precursor of platinum having an oxidation state II is selected from PtCl₂, (Bipy)PtCl₂ and Pt(NH₃)₄Cl₂.

15. The process according to any one of claims 11 to 14 **characterised in that** the compound of platinum having an oxidation state II is selected from (Bipy)PtCl₂ and Pt(NH₃)₄Cl₂.

16. The process according to any one of claims 11 to 15 **characterised in that** the metal ion(s) M in the precursor of the metal ion(s) is Ag⁺.

17. The process according to any one of claims 11 to 16 **characterised in that** the heteropolyanion is K₆P₂W₁₈O₆₂.

18. The process according to any one of claims 11 to 17 **characterised in that** the electron-conducting material is a titanium metal sponge or a carbon felt or fabric.

19. A fuel cell **characterised in that** it comprises a catalyst according to any one of claims 1 and 3 to 6.

20. A fuel cell **characterised in that** it comprises an electrode according to either of claims 9 or 10, or an electrode obtained by the process according to any one of claims 11 to 18.
